# FASCICULE DE BREVET EUROPEEN

(11) **EP 0 563 255 B1**
(45) Date de publication et mention de la délivrance du brevet: **28.09.1994**
(21) Numéro de dépôt: 92903034.4
(22) Date de dépôt: 20.12.1991
(51) Int. Cl.: C12N 15/37, C12P 21/02, C07K 15/00, C12Q 1/68, G01N 33/567

(54) **SEQUENCES D'ADN DERIVEES DU GENOME DU PAPILLOMAVIRUS HPV39, LEUR APPLICATION AU DIAGNOSTIC IN VITRO ET A LA PRODUCTION DE COMPOSITION IMMUNOGENE**
VOM GENOM DES PAPILLOMAVIRUS-HPV-39 ABGELEITETE DNS-SEQUENZEN, DEREN ANWENDUNG IN DER IN VITRO-DIAGNOSE UND ZUR HERSTELLUNG EINER IMMUNOGENENZUSAMMENSETZUNG
HPV39 PAPILLOMAVIRUS GENOME-DERIVED DNA SEQUENCES, THEIR APPLICATION TO IN VITRO DIAGNOSIS AND PRODUCTION OF IMMUNOGENIC COMPOSITIONS

(30) Priorité: 20.12.1990 FR 9016044
(43) Date de publication de la demande: 06.10.1993
(73) Titulaire: INSTITUT PASTEUR, F-75724 Paris Cédex 15 (FR); INSTITUT NATIONAL DE LA SANTE ET DE LA RECHERCHE MEDICALE (INSERM), F-75654 Paris Cédex 13 (FR)
(72) Inventeur: ORTH, Gérard, F-92330 Sceaux (FR); VOLPERS, Christoph, D-6200 Wiesbaden (DE); STREECK, Rolf, E., D-65189 WIESBADEN (DE)
(74) Mandataire: Desaix, Anne
(86) Numéro de dépôt international: FR9101053
(87) Numéro de publication internationale: WO9211369

(56) Documents cités:
- EP-A- 0 235 004
- EP-A- 0 375 555

## Description

L'invention concerne des séquences d'ADN déterminées dérivées du génome du papillomavirus HPV39, y inclue la séquence correspondante à son génome entier, ainsi que des ADNs recombinants, notamment vecteurs contenant ces séquences d'ADN. L'invention concerne également les cultures cellulaires transformées avec les dits ADNs recombinants dans des conditions leur permettant d'exprimer les séquences correspondantes dérivées du génome de HPV39 sous la forme des protéines correspondantes. L'invention concerne enfin les protéines, elles-mêmes purifiées, telles qu'obtenues à partir de ces cultures cellulaires. Enfin, l'invention est relative à la production des compositions immunogènes contenant de telles protéines ou fragments de protéines.

La suite de l'exposé sera faite par référence aux figures ci-jointes, et dont les légendes suivent :
Fig.1 : Séquence nucléotidique du brin d'ADN d'HPV39 analogue à l'ARNm. La position 1 sur le génome circulaire a été déterminée par alignement avec HPV16 et 18.
Fig.2 : Distribution des codons de départ (start) (barres au dessus) et des codons stop (barres au dessous) dans les trois cadres de lecture des deux brins d'ADN d'HPV39. (a) brin analogue à l'ARNm (b) brin complémentaire. Les ORFs dans (a) ont été identifiés par comparaison avec d'autres types d'HPV. La numérotation est en accord avec celle de la Figure 1.

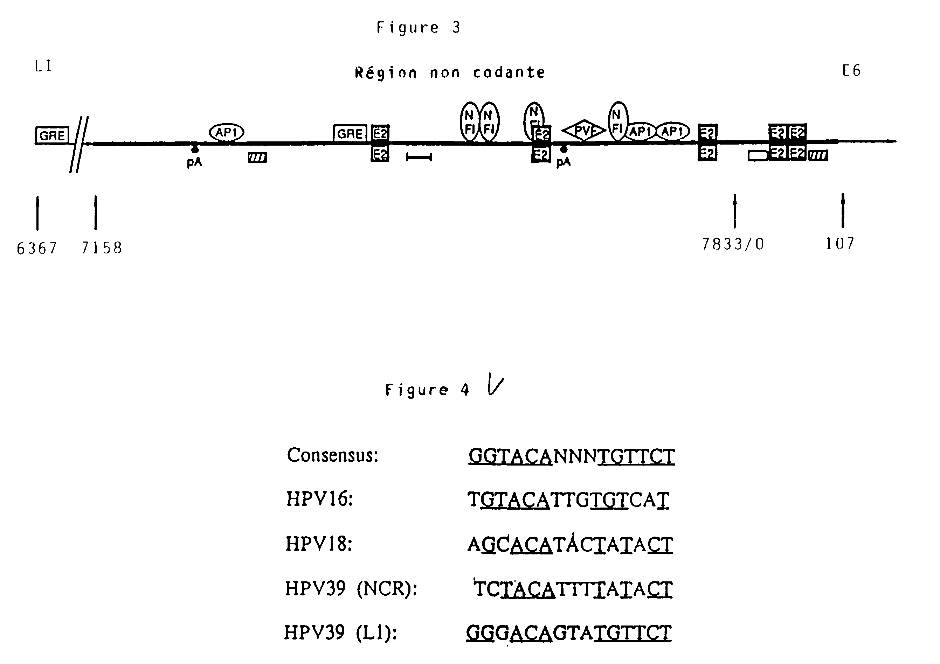

Fig.3 : Caractéristiques principales de la région non-codante. Les motifs de séquences suivants de la NCR sont présentés à partir du nucléotide 7158 jusqu'au nucléotide 106 : représente le palindrome de 12 bp spécifique du papillomavirus (les second et troisième motifs sont dégénérés), représente le site de polyadénylation, représente la boite TATA, représente le présumé élément promoteur, représente l'élément de réponse glucocorticoïde, représente la séquence amplificatrice du noyau, représente le probable site de liaison du facteur nucléaire I, représente le site présumé de liaison de la protéine activatrice 1, représente le site de liaison du facteur associé à l'amplificateur de papillomavirus.
Fig.4 : Comparaison des éléments probables de réponse glucocorticoïde (GREs) d'HPV39 comparés aux GREs de HPV16 et 18 et à une séquence consensus GRE/PRE. Les nucléotides identiques à la séquence consensus sont soulignés.

L'invention repose sur la découverte de séquences particulières présentes dans HPV39, séquences qui en font l'originalité et qui permettent des détections particulièrement fines de papillomavirus du type HPV39. Ces séquences ou des fragments de ces séquences sont utilisables pour la constitution de sonde d'hybridations particulièrement sensibles, en particulier d'amorces permettant des analyses par la méthode dite de PCR. Avant de procéder plus avant à la description de ces séquences ou fragments de séquences, il est proposé de faire un bref retour sur l'état de la technique et, ensuite, de fournir une description détaillée du génome de HPV39.

Parmi les 60 types différents de papillomavirus humains sinon davantage, certains sont associés aux néoplasies et aux carcinomes de l'appareil génital (1). L'ADN de HPV16 et HPV18 ont été détectés dans 50% et 20% des biopsies de cancers cervicaux, vulvaire ou pénien (2,3). Les HPV31, 33, 35, 39 et 45 ont été moins fréquemment rencontrés dans de telles lésions (1, 4). En utilisant l'ADN de HPV6 en tant que sonde d'hybridation dans des conditions de faible stringence, HPV39 a tout d'abord été cloné à partir d'échantillons de biopsies de papules péniennes Bowenoïdes, qui contiennent l'ADN viral sous sa forme épisomale. Par contre l'ADN viral a été retrouvé intégré à l'intérieur du génome cellulaire de carcinomes invasifs (5). Une étude récente réalisée à partir de 365 patientes infectées par HPV, l'ADN de HPV39 a été détecté dans 3,9% des échantillons de tissu.

L'étude biologique de HPV a été entravée par l'absence d'un système de culture de tissu pour la propagation virale in vitro. L'analyse de la séquence d'un certain nombre de génomes de papillomavirus (2, 3, 6-14) a constitué la base pour la compréhension de leur organisation génétique et de leur régulation, pour l'expression de gènes individuels et la génération d'antisérums, et pour l'évaluation des relations phylogénétiques parmi les nombreux types de HPV.

La séquence nucléotidique complète du génome de HPV39 a été déterminée. L'ADN de HPV39 isolé à partir du clone d'origine (5) a été souscloné dans pUC18 en trois fragments de taille similaire en utilisant le site unique EcoRI et les deux sites BamHI. Des clones appropriés pour le séquençage d'un brin d'ADN ont été générés en créant des séries de détections à l'aide de l'éxonucléase III selon la procédure de Henikoff (15). Le second brin a été séquencé en utilisant des oligonucléotides synthétiques complémentaires du premier brin en tant qu'amorces. Le séquençage du plasmide double brins a été réalisé en utilisant la méthode de terminaison de chaîne didéoxy de Sanger et al. (16, 17).

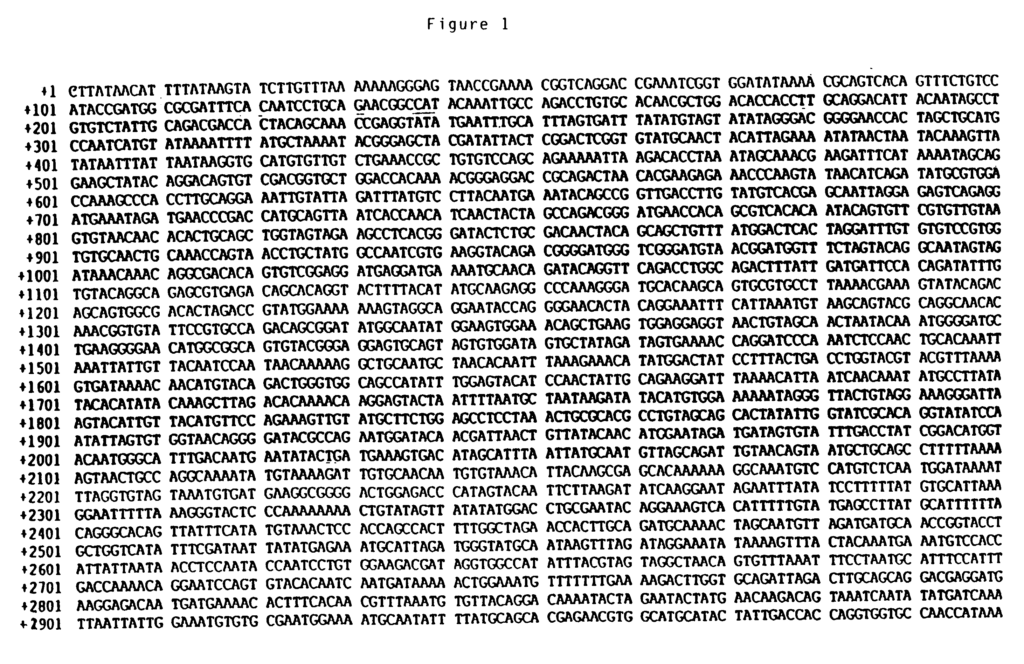

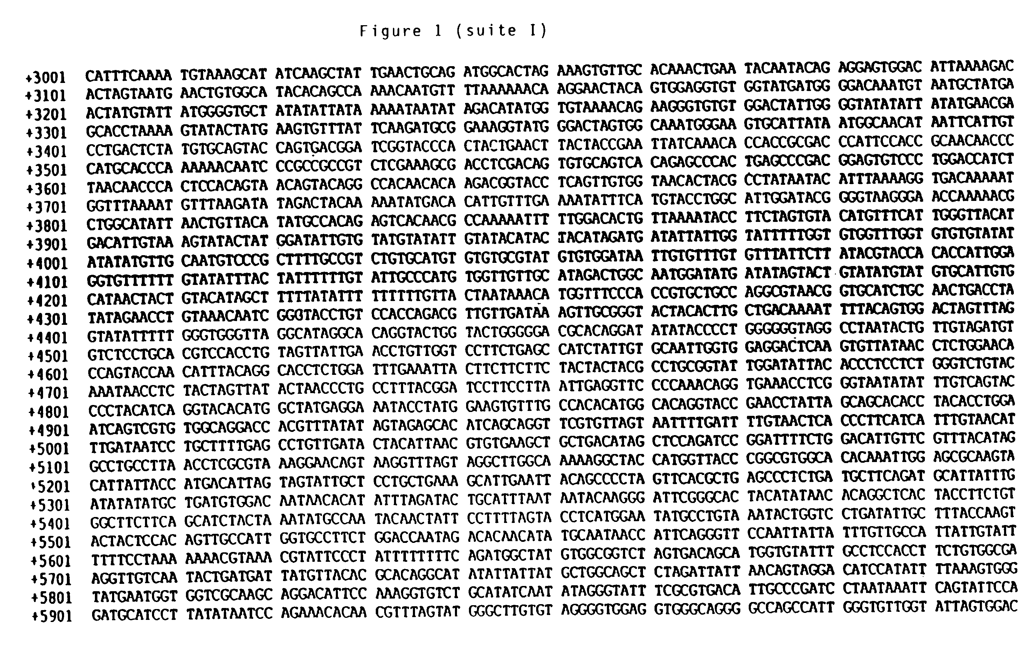

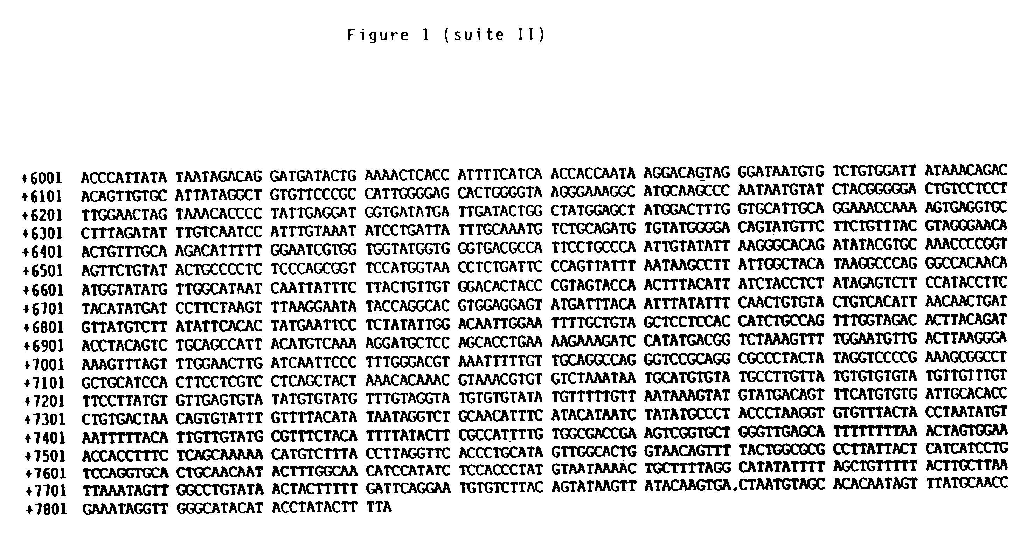

L'ADN de HPV39 comprend 7833 bp (fig. 1) et présente un contenu G/C de 40%. La carte de restriction dérivant de la séquence a été confirmée par digestion de l'ADN. Elle est en accord avec la carte de restriction publiée par Beaudenon et al. (5) à l'exception d'un site supplémentaire HindII, d'un site supplémentaire PvuII et de quatre sites additionnels AvaII. Un site AavaII (à la position 3592) est résistant au clivage après clonage dans E.coli en raison d'un chevauchement avec la séquence de reconnaissance de la méthylase dcm de E.coli.

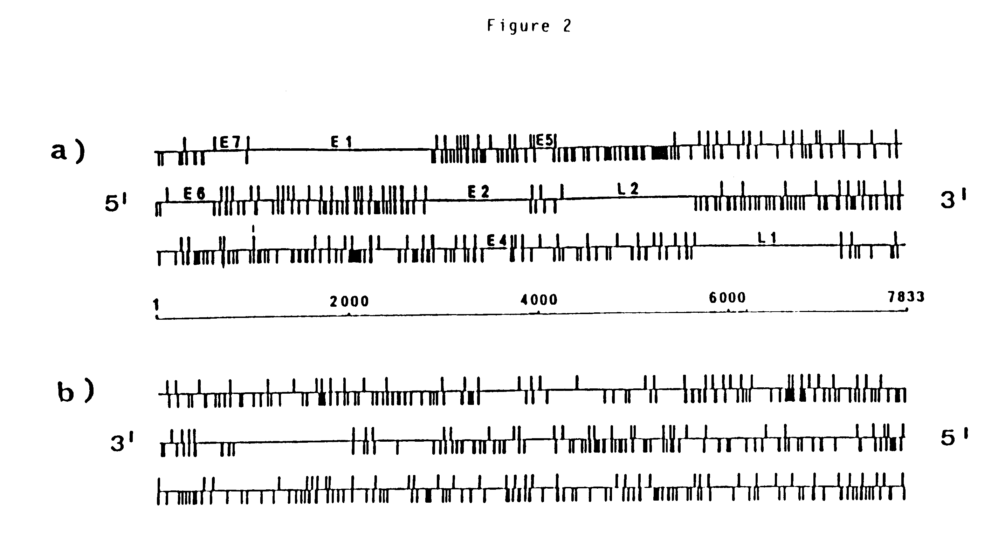

HPV39 présente un ensemble de phases de lecture ouvertes (Open Reading Frames: ORFs) conservées chez tous les HPVs séquencés jusqu'à maintenant (Fig.2a, tableau 1). Les ORFs codant pour les protèines supposées précoces et pour les composants de la capside, sont localisés sur le même brin d'ADN ; ils sont séparés par une région non-codante (Non coding region: NCR) de 782 paires de base (base pairs: bp). Les caractéristiques génomiques partagées par HPV39 avec d'autres papillomavirus génitaux sont représentés par les chevauchements entre les ORFs E1 et E2, L2 et L1, l'inclusion de E4 à l'intérieur de E2, la localisation de E7 immédiatement en amont de E1 (3) et l'absence du codon d'initiation ATG dans E4, comme il a été précédemment décrit pour HPV16, 31 et 33 (2,4,12).

Les brins d'ADN complémentaires de tous les génomes séquencés jusqu'à maintenant ne contiennent pas de ORFs excédant une taille de 0,6 à 0,8 kb. La signification fonctionnelle de tel ORFs a été démentie par l'absence de toute séquence de régulation détectable. De plus, il n'a pu être mis en évidence qu'ils étaient transcrits dans les cellules transformées par HPV ou affectées par BPV (7, 9, 18, 19). Un grand ORF de 1,3 kb a maintenant été trouvé sur ce brin d'ADN de HPV39 (nucl. 2050-776), qui comprend un codon ATG et un site accepteur potentiel d'épissage (GCTGCTACAGG, nucl. 1871-1861) proche de l'extrémité 5' (Fig.2b). Plus loin en amont du même brin d'ADN, un ORF mineur (nucl. 4204-3875) est précédé d'une séquence TATA à une distance de 23 bp (nucl. 4227) et d'un site de liaison pour le facteur nucléaire I (nucl. 4271) (20,21). A l'extrémité 3' de l'ORF de 1,3 kb, un signal de polyadénylation AATAAA (nucl.411) pourrait servir pour la maturation d'un produit de transcription primaire (22). D'autres expériences devraient permettre de savoir si cet ORF est transcrit in vivo.

La région non-codante HPV39 est divisée en trois segments par trois versions complètes et deux versions dégénérées du palindrome ACCGNNNNCGGT spécifique des papillomavirvs (nucl. 43, 59, 7456, 7625, 7798), un motif amplificateur (enhancer) E2-dépendant (23,24). Ces segments correspondent à des sections de taille similaire dans les génomes de HPV16, 18 et 33 (3). Il y a deux séquences "de boite" TATA (TATA box sequences) dans la région non-codante, l'une d'entre elle étant localisée en amont de l'ORF E6. Cette dernière constitute vraisemblablement une partie du promoteur E6 et d'autres gènes précoces, en association avec un promoteur conservé AAAGGGAGTA placé en amont des séquences répétées en tandem du palindrome de 12 bp (25-27). Le site de polyadénylation présumé pour les produits de transcription virale tardive est localisé à un emplacement situé à environ 100 paires de base en aval du codon stop L1. Un autre élément AATAAA situé en aval de l'ORF E5 pourrait servir de signal de polyadénylation pour les produits de transcription précoce (18).

Les longues régions de contrôle des HPVs génitaux précédemment séquencés contiennent des sites de liaisons pour de nombreux facteurs de transcription et ont été présentées comme fonctionnant en temps qu'amplificateurs spécifiques de type cellulaire pour HPV6, 11, 16 et 18 (28-30). La région de régulation de HPV39 contient quatre sites de liaisons possibles pour le facteur nucléaire I (NFI) (21, 31), deux pour la protéine d'activation 1 (AP1) (21,32) un motif de liaison pour "le facteur associé à l'amplificateur de papillomavirus" récemment décrit (papillomavirus enhancer associated factor: PVF) (31), et une "séquence amplificatrice du noyau" conservée dans SV40 et dans d'autres virus (33) (Fig.3). Un élément potentiel de réponse glucocorticoïde (glucoprotein reponse element: GRE) ressemble à ceux qui se trouvent dans les NCRs de types HPV apparentés (27). Un élément GRE supplémentaire hautement conservé trouvé dans l'ORF (nucl. 6367) n'a pas d'équivalent dans d'autres types d'HPV (Fig.4). Il est en chevauchement avec un site de liaison AP2 que l'on retrouve également dans les amplificateurs de SV40 et BPV (34). Cela indique la présence d'une fonction de régulation pour la région située en amont de la NCR. Un effet coopératif de NFI et de AP1, ainsi que de NP1 avec le récepteur glucocorticoïde, respectivement, a été décrit pour l'amplificateur de HPV16 (30). L'interaction de nombreux facteurs avec les sites de liaisons potentiels dans la NCR et dans L1 de HPV39 doit encore être élucidé. Des sites donneurs d'épissage (nucl.233) et accepteurs (nucl.408) sont localisés en aval de la NCR. Ils ont été démontrés comme étant importants pour la génération de l'ARNm E7 dans les types d'HPV oncogènes (4,35).

Des comparaisons de séquences d'ORFs individuels indiquent que HPV39 a le plus haut degré d'homologie avec HPV18. Il est apparenté de façon plus distante à d'autres papillomavirus génitaux, et encore moins à un membre des types de HPVs cutanés, HPV8 (Tableau 2). Ainsi HPV39 appartient à un sous-groupe d'HPVs génitaux présentant un potentiel oncongène considérable incluant HPV18, HPV45(1) et un nouveau type présentant une forte homologie avec HPV39 récemment cloné à partir d'une lignée cellulaire du carcinome ME180 distinct du groupe HPV16/31/33.

Les ORFs E6 et E7 sont habituellement transcrits dans les lignées cellulaires du cancer cervical et du carcinome (36), et les produits de leurs gènes sont impliqués dans l'immortalisation et la transformation des cellules épithéliales primaires et des fibroblastes (37). Quatre motifs Cys-X-X-Cys dans la protéine E6 de tous les papillomavirus séquencés qui pourraient être impliqués dans la liaison de l'ADN par formation de structures du type "doigt de zinc" ("zinc finger") (38), sont également présents dans HPV39. Par contre, seulement le premier des deux éléments bien conservés Cys-X-X-Cys peut être retrouvé dans la protéine E7. Le second élément a une cystéine substituée par une tyrosine. Comme l'analyse mutationnelle de l'ORF E7 de HPV16 a permis de montrer qu'un doigt de zinc ("zinc finger") est suffisant pour la capacité de transformation (39), la protéine E7 de HPV39 demeure probablement fonctionnelle. De plus, la protéine E7 de HPV39 contient un motif "protéine de division cellulaire (cd)" qui est commun à de nombreux produits de gènes impliqués dans la transformation, comme l'antigène T de SV40, E1A de l'adénovirus, la protéine myc et les protéines E7 des HPVs génitaux malins, mais pas de HPV6 ni HPV11 (4) :
asp/asn-x-x-cys-x-ser/thr/glu-x-(1-8)-asp/glu-asp/glu/ser/thr-asp/glu (les acides aminés de E7 de HPV39 sont soulignés). Les motifs cd du grand antigène T de SV40 et de la protéine de l'adénovirus semblent être responsables de la liaison du produit antioncogène du rétinoblastome (40). L'activité de transformation de E7 peut également être attribuée à une interaction protéine-protéine via la séquence cd.

L'invention concerne plus particulièrement la séquence correspondante au grand ORF de 1,3 kb s'étendant du nucléotide 2050 au nucléotide 776 de la Figure 1 ou à tout fragment contenu dans cette séquence ou qui en serait dérivé, ce fragment comportant au moins 15 nucléotides.

L'invention concerne également encore plus particulièrement la séquence correspondante à l'ORF mineur s'étendant du nucléotide 4204 au nucléotide 3875 et voir même du nucléotide 4227 au nucléotide 3875 ou des séquences issues ou dérivées de ces dernières.

De même l'invention concerne encore des séquences d'au moins 15 nucléotides issus ou dérivés de la séquence totale de HPV39 et comportant au moins 15 nucléotides et contenant l'élément GRE hautement conservé comportant la séquence GACA incluse dans l'élément GRE hautement conservé au niveau du nécléotide 6367 et plus particulièrement visée à la Figure 4. Enfin, plus spécifiquement l'invention concerne les 3 séquences nucléotidiques identifiées ci-après :
- Consensus :: GGTACANNNTGTTCT
- HPV39 (NCR) :: TCTACATTTTATACT
- HPV39 (L1) :: GGGACAGTATGTTCT
Elle concerne encore tous fragments dont les tailles n'excèdent pas celles des fragments précédemment définis, les premiers étant caractérisés en ce qu'ils hybrident avec les seconds dans des conditions strictes (Tm -20°C), notamment lorsque l'on opère dans les conditions suivantes :
L'hybridation est effectuée à 42°C dans une solution contenant : 50 mM de tampon phosphate de sodium pH = 6,5 ; 5xSSC (1xxSSC = 0,15 M NaCL, 0,015 M Na Citrate) ; 50% de formamide ; 200 ug/ml d'ARN de transfert de levure ; 0,02% de solution de Denhart.

Font encore partie de l'invention les fragments appartenant aux mêmes types, notamment en ce qu'ils présentent des pourcentages d'hybridations croisées supérieurs à 50% avec les fragments définis plus spécifiquement plus haut.

L'utilisation de l'une ou l'autre des séquences sus-indiquées ou d'acides nucléiques les contenant sont particulièrement apporpriés à la réalisation de sonde d'hybridation permettant la détection dans un échantillon biologique d'ADN papillomavirus apparenté au HPV39.

D'une façon générale, l'invention concerne donc également tout ADN recombinant contenant l'ADN-HPV susdit ou des fragments de cet ADN-HPV, en particulier des sondes d'hybridation formées de ces ADNs recombinants et spécialement adaptées à la détection d'une infection par HPV39 ou d'un variant ou sous-type de ce papillomavirus. Ces sondes peuvent, soit être marquées elles-mêmes, soit être modifiées au niveau de certains nucléotides, notamment en vue de leur couplage, direct ou indirect, avec un marqueur distinct. Il va de soi que dans ces sondes les parties étrangères à la séquence nucléotidique correspondante à l'ADN du papillomavirus et provenant normalement d'un vecteur de clonage, sont telles qu'elles ne risquent pas d'hybrider dans des conditions stringentes avec les autres acides nucléiques éventuellement contenus dans l'échantillon testé pour sa teneur éventuelle en ADN du papillomavirus correspondant ou de l'un de ses variants.

Le procédé selon l'invention pour le diagnostic in vitro sur un échantillon biologique à tester, provenant normalement d'un patient humain, d'une infection par un papillomavirus pouvant entraîner ou ayant entraîné une néoplasie génitale, notamment un cancer cervical, vulvaire ou pénien, est donc caractérisé par la mise en contact d'une sonde telle que ci-dessus définie, avec les acides nucléiques de cet échantillon, le cas échéant préalablement rendus accessibles à la sonde, de préférence dans des conditions d'hybridation stringentes, et par la détection de l'hybride formé entre l'ADN viral recherché, éventuellement présent dans l'échantillon et ladite sonde.

Chacune des sondes selon l'invention ou les mélanges contenant la susdite sonde peuvent notamment être utilisés comme suit, étant naturellement entendu que les essais de diagnostic décrits ne sauraient être considérés comme limitatifs des conditions d'emploi dans lesquelles ces sondes ou mélanges de sondes peuvent en fait être utilisés.

Dans l'exemple considéré, il s'agit par exemple d'identifier un HPV dans une biopsie, dans des cellules obtenues par grattage de lésions, ou dans des coupes de biopsies fixées par le mélange de Carnoy (éthanol, chloroforme, acide acétique 6:3:1) et incluses dans la paraffine. L'Examen nécessite l'extraction préalable de l'ADN des prélèvements selon des méthodes dont le principe est connu et met en jeu l'analyse de cet ADN par des expériences d'hybridation moléculaire, effectuées dans des conditions strictes ou moins strictes, à l'aide de sondes radioactives (marquées au ³² p ou au ³⁵ S) préparées à partir de l'HPV selon l'invention ou de mélanges d'ADNs ou d'HRVs le contenant.

Plusieurs méthodes d'hybridation peuvent être utilisées. On peut, par exemple, mettre en oeuvre la méthode d'hybridation sur tache. Cette méthode comporte, après dénaturation de l'ADN, le dépôt d'une quantité aliquote d'ADN sur des membranes (nitrocellulose ou "Genescreenplus", l'hybridation de chaque membrane, dans les conditions usuelles, avec un mélange de sondes et la détection des hybrides radioactifs, par exposition des membranes au contact d'un film radiographique. On peut aussi utiliser une méthode d'hybridation sur réplique. Cette méthode comprend la séparation électrophorétique en gel d'agarose des fragments d'ADN engendrés après traitement de l'ADN par des enzymes de restriction, le transfert des fragments, après dénaturation alcaline, sur des membranes (nitrocellulose, "Genescreenplus") et leur hybridation, dans les conditions usuelles, avec le mélange approprié de sondes. La formation d'hybrides radioactifs est détectée après exposition des membranes au contact d'un film radiographique.

Les sondes radioactives sont constituées soit par des ADNs d'HPVs marqués par la méthode de translation de coupure ("nick-translation"), soit par des ARNs préparés par transcription d'ADNs viraux insérés dans un vecteur, par exemple de type SP6. L'utilisation de sondes radioactives présente l'avantage d'une grande sensibilité, mais ceci n'exclut pas l'utilisation de sondes non radioactives, par exemple de sondes biotinylées et susceptibles d'être reconnues par des anticorps soit marqués eux-mêmes, soit eux-mêmes reconnus par des anticorps portant un marqueur enzymatique, fluorescent, etc...

L'invention concerne également des cultures cellulaires compétentes transformées avec des ADNs recombinants du type sus-indiqué, en particulier ceux dans lesquels la séquence nucléotidique correspondant à l'ADN ou la séquence de l'ADN de HPV39 est placée sous le contrôle d'éléments de transcription et de régulation de cette séquence nucléotidique dans ladite culture cellulaire.

Par conséquent, elle concerne également les produits d'expression de ces ADNs recombinants dans les hôtes cellulaires compétents correspondants et les anticorps correspondants susceptibles d'être produits contre ces produits d'expression.

A ce titre, l'invention concerne les polypeptides résultant notamment des expressions respectives des gènes E1, E2, E4, E6, E7, L1, L2 de l'ADN-HPV39.

Le procédé selon l'invention pour la production de ces polypeptides comprend par conséquent la transformation de cultures cellulaires compétentes avec les ADNs recombinants contenant les séquences nucléotidiques correspondantes issues de HPV39, de façon telle que la séquence nucléotidique correspondant à l'une desdites protéines puisse être exprimée dans cet hôte cellulaire, la récupération de ces polypeptides à partir des produits synthétisés par l'hôte cellulaire compétent et la purification (par exemple par mise en contact des produits d'expression préalablement extraits des cultures cellulaires ou du milieu dans lequel celles-ci ont été développées, avec des anticorps préalablement formés contre de tels polypeptides).

Les produits d'expression des séquences L2 de chacune des génomes des papillomavirus selon l'invention présentent cependant un intérêt tout particulier, en ce qu'ils peuvent eux-mêmes être utilisés pour la production in vivo d'anticorps susceptibles de reconnaître les produits d'expression du gène L2 dans des prélèvements biologiques affectés par un papillomavirus du type HPV39 ou d'un variant de celui-ci, et ce plus particulièrement lorsque les préparations du genre en question ont préalablement été fixées.

L'invention concerne encore des polypeptides hybrides contenant les polypeptides susdits et dérivés respectivement de HPV39, par exemple la protéine L2 fusionnée à d'autres séquences polypeptidiques, dans la mesure où celles-ci ne modifient pas de façon essentielle les propriétés immunogènes de la protéine L2. La présence de ces autres fragments polypeptidiques peut notamment résulter du mode de production utilisé de ces polypeptides hybrides, par exemple par génie génétique. Par exemple, ces polypeptides hybrides contiennent une séquence dérivée de la β-galactosidase. De tels produits peuvent notamment être obtenus par transformation de E. coli avec des vecteurs appropriés (phages ou plasmides) modifiés par tout ou partie de l'opéron lactose et comportant en outre, insérée en aval du promoteur de l'opéron-lactose (ou de tout autre promoteur approprié, par exemple de phage λ), la séquence nucléotidique dérivée du gène L2 issu de HPV39. On a avantageusement recours à des plasmides ou phages de ce type comprenant une partie au moins du gène de la β-galactosidase de l'opéron-lactose.

Les polypeptides selon l'invention peuvent également, lorsqu'ils ont été purifiés, être mis en oeuvre dans des techniques de purification des anticorps qui leur correspondent, notamment à partir de sérums d'animaux qui avaient été immunisés par ces polypeptides. En particulier, ces polypeptides peuvent ête fixés sur des colonnes d'affinité. Les opérations de purification des anticorps consistent alors à faire passer le sérum les contenant au contact de colonnes d'affinité portant les susdits polypeptides. Les anticorps sélectivement fixés sur ces colonnes peuvent ensuite être récupérés par dissociation des complexes antigènes-anticorps, au moyen d'un tampon approprié, présentant une force ionique adéquate, par exemple une solution aqueuse d'un sel tel que l'acétate d'ammonium. On peut également avoir recours à des solutions acidifiées.

L'invention concerne également un procédé de production d'anticorps contre lesdits polypeptides, en particulier contre les produits d'expression des gènes E6, E7 ou, de préférence L2 de HPV39, ce procédé comprenant l'immunisation d'un hôte vivant approprié avec lesdits polypeptides et la récupération des anticorps formés à partir d'un sérum de l'hôte immunisé, notamment par mise en contact de ces sérums avc les polypeptides correspondant à l'état purifié et par la récupération de ces anticorps à partir des complexes antigène-anticorps formés.

En particulier, l'invention concerne les anticorps susdits, préalablement purifiés, en association avec un véhicule pharmaceutique approprié. Cette composition est alors susceptible d'être mise en oeuvre pour le traitement de l'affection donnée, dès lors que celle-ci aurait été diagnostiquée cliniquement, à l'issue d'un essai de diagnostic in vitro sur un prélèvement histologique ou cytologique provenant du malade. Cette composition (notamment sous forme de sérum) peut être administrée, de préférence par voie parentérale. Ce sérum est alors susceptible de provoquer une régression des infections induites par des papillomavirus du type HPV39.

Ces anticorps peuvent plus particulièrement être mis en oeuvre dans des essais de diagnostic d'une infection due à HPV39 ou à un papillomavirus apparanté (ou à un variant de ce papillomavirus), dans la mesure où des coupes histologiques provenant des personnes infectées peuvent également contenir des produits d'expression de certains de ses gènes de structure, notamment L2.

L'invention concerne donc encore un procédé de diagnostic in vitro, en particulier de néoplasies génitales des cancers cervicaux, vulvaires ou péniens, comprenant la mise en contact de coupes histopathologiques provenant de lésions induites chez les personnes concernées dans des conditions permettant la production d'un complexe antigène-anticorps et la détection de ce complexe antigène-anticorps. Avantageusement, la détection se fait sur des préparations fixées au préalable dans des conditions dissociantes, par exemple avec le milieu ou mélange de CARNOY déjà mentionné plus haut (également décrit dans l'ouvrage de L. LISON, intitulé "Histochimie et cytochimie animales").

Les anticorps anti-L2 éventuellement fixés peuvent être reconnus par d'autres anticorps formés contre les premiers, ces autres anticorps portant des marqueurs appropriés, de préférence non radioactifs. Ces marqueurs sont par exemple de nature enzymatique ou fluorescente.

Les anticorps ainsi sélectionnés, comme les sondes d'hybridation sus-définies qui leur correspondent, peuvent par conséquent être utilisées pour diagnostiquer in vitro les types d'affections correspondants.

L'invention concerne enfin les compositions vaccinantes correspondantes contenant une ou de préférence plusieurs autres protéines L2, en association avec un véhicule pharmaceutiquement acceptable adapté au mode d'administration choisi, notamment par voie parentérale. Ces compositions sont utilisables pour protéger les personnes soumises à de hauts risques d'infection par HPV39 ou par des papillomavirus de type correspondant.

**TABLEAU 1**

| CADRES OUVERTS DE LECTURE (EN ANGLAIS "OPEN READING FRAMES" ABBREVIATION DANS "ORF") LE BRIN ANALOGUE D'ARNm DU GENOME DE HPV39 | | | | | |
|---|---|---|---|---|---|
| ORF | Premier nucléotide | Premier ATG | Nucléotide précédent le codon d'arret | ORF taille (pb) | Poids moléculaire (kD) |
| E6 | 44 | 107 | 580 | 537 | 18.7 |
| E7 | 493 | 592 | 918 | 426 | 12.5 |
| E1 | 922 | 928 | 2868 | 1947 | 73.0 |
| E2 | 2780 | 2798 | 3907 | 1128 | 43.0 |
| E4 | 3393 | - | 3674 | 282 | - |
| E5 | 3958 | 3958 | 4173 | 216 | 9.0 |
| L2 | 4172 | 4250 | 5659 | 1488 | 49.9 |
| L1 | 5610 | 5643 | 7157 | 1548 | 56.0 |

**TABLEAU 2**

| COMPARAISON DE PROTEINES HPV AVEC HPV39 (POURCENTAGE D'ACIDES AMINES IDENTIQUES)^{a} | | | | | |
|---|---|---|---|---|---|
| | HPV18 | HPV33 | HPV16 | HPV11 | HPV8 |
| E6 | 68% | 51% | 48% | 38% | 26% |
| E1 | 71% | 55% | 48% | 51% | 40% |
| E2 | 53% | 47% | 49% | 47% | 33% |
| L2 | 71% | 53% | 56% | 50% | 37% |
| L1 | 77% | 66% | 65% | 65% | 54% |

| | | | | | |
|---|---|---|---|---|---|
| ^{a} Les comparaisons de séquences ont été réalisées en ayant recours à un programme d'ordinateur basé sur un algorythme de Needlemann et Wunsch (41). | | | | | |

### REFERENCES

1. De Villiers. E., J. Virol. 63, 4898-4903 (1989).
2. Seedorf, K.,Krämmer, G.,Dürst, M.,Suhai, S. and Röwekamp, W.G., Virology 145, 181-185 (1985).
3. Cole, S.T. and Danos. O.,J. Mol. Biol. 193, 599-608 (1987).
4. Goldsborough, M.D.,DiSilvestre. D., Temple, G.F. and Lorincz. A.T., Virology 171, 306-311 (1989).
5. Beaudenon, S., Kremsdorf. D., Obalek, S.,Jablonska, S., Pehau-Amaudet, G., Croissant, O. and Orth, G., Virology 161,374-384 (1987).
6. Danos, O., Katinka. M. and Yaniv, M., EMBO J. 1, 231-236 (1982).
7. Chen. E., Howley, P.M., Levinson, A.D., Seeburg, P.H., Nature 299, 529-534 (1982).
8. Dartmann, K.,Schwarz.E., Gissmann, L. and Zur Hausen, H., Virology 151,124-130(1986).
9. Fuchs, P.G., Hiner. T., Weninger, J. and Pfister, H.,J. Virol. 58, 626-634 (1986).
10. Giri, I., Danos, O. and Yaniv, M., Proc. Natl. Acad. Sci. USA 82, 1580-1584 (1985).
11. Groff, D.E. and Lancaster, W.D.,J. Virol. 56, 85-91 (1985).
12. Cole, S.T. and Streeck, R.E., J. Virol. 58, 991-995 (1986).
13. Schwarz, E.,Dürst, M.,Demankowski, C.,Lattermann, O.,Zech, R., Wolfsperger, E., Suhai, S. and Zur Hausen, H., EMBO J. 2,2341-2348 (1983).
14. Zachow, K.R., Ostrow, R.S., Faras, A.J.,Virology 158, 251-254 (1987).
15. Henikoff, S.,Methods in Enzymology 155,156-165 (1987).
16. Sanger, F.,Nickeln, S. and Coulson, A.R., Proc. Natl. Acad. Sci. USA 74, 5463-5467 (1977).
17. Zhang, H., Scholl, R., Browse, J. and Somerville, C., Nucl. Acids Res. 16, 1220 (1988).
18. Chow, L.T.,Nassen, M., Wolinsky, S.M. and Broker, T.R.,J. Virol. 61, 2581-2588 (1987).
19. Engel, L.W., Heilman, C.A. and Howley, P.M.,J. Virol. 47, 516-528 (1983).
20. Benoist, C. and Chambon, P., Nature 290, 304-310 (1981).
21. Wingender, E,. Nucl. Acids Res. 16, 1879-1902 (1988).
22. Proudfoot, N.,Nature 298, 516-517 (1982).
23. Spaiholz, B.A., Yang, Y. and Howley, P.M.,Cell 42, 183-191 (1985).
24. Hirochika, H., Broker, T.R. and Chow, L.T.,J. Virol. 61, 2599-2606 (1987).
25. Gloss, B., Chong, T. and Bernard, H.-U.,J. Virol. 63, 1142-1152 (1989).
26. Thierry, F., Heard. J.M., Dartman, K. and Yaniv, M., J. Virol. 61, 134-142 (1987).
27. Chan, W., Klock, G. and Bernard, H.-U.,J. Virol. 63, 3261-3269 (1989).
28. Chin, M.T., Broker, T.R. and Chow, L.T.,J. Virol. 63, 2967-2976 (1989).
29. Cripe, T.P., Haugen, T.H., Turk, J.P., Tabatabai, F., Schmid, P.G., Dürst, M., Gissmann, L., Roman, A. and Turek, L.P., EMBO J. 6, 3745-3753 (1987).
30. Chong, T., Chan, W.K. and Bernard, H.-U.,Nucl. Acids. Res. 18, 465-470 (1990).
31. Gloss, B., Yeo-Gloss, M., Meisterernst, M., Rogge, L., Winnacker, E.L. and Bernard, H.-U., Nucl. Acids. Res. 17, 3519-3533 (1989).
32. Angel, P., Imagawa, M., Chin, R., Stein, B., Imbra, R.J., Rahmsdorf, H.J., Jonat, C., Herrlich, P. and Karin, M., Cell 49, 729-738 (1987).
33. Weiher, H., König, M. and Gruss, P., Science 219, 626-631 (1983).
34. Imagawa, M., Chin, R. and Karin, M., Cell 51, 251-258 (1987).
35. Smotkin, D., Prokoph, H. and Wettstein, F.O.,J. Virol. 63, 1441-1447 (1989).
36. Smotkin, D. and Wettstein, F.O., Proc. Natl. Acad. Sci. USA 83, 4680-4684 (1986).
37. Münger, K., Phelps, W.C.,Bubb, V.,Howiey, P.M. and Schlegel, R.,J. Virol. 63, 4417-4421 (1989).
38. Evans, R.M. and Hollenberg, S.M., Cell 52, 1-3 (1988).
39. Edmonds, C. and Vousden, K.H.,J. Viroi. 63. 2650-2656 (1989).
40. Figge, J. and Smith. T.F., Nature 334, 109 (1988).
41. Needleman, S.B. and Wunsch, C.D.,J. Mol. Biol. 48, 443-450 (1970).

## Revendications

1. ADN d'un papillomavirus HPV39 caractérisé, soit par la séquence de nucléotides de la Figure 1, soit par une partie de cette séquence mais comportant au moins quinze nucléotides, soit encore par une séquence permettant à cet ADN de s'hybrider avec l'ADN de HPV39 dans des conditions stricles, la susdite partie de séquence étant :
- soit incluse dans celle du grand ORF de 1,3 kb s'étendant du nucléotide 2050 au nucléotide 776 de la figure 1 ;
- soit incluse dans celle de l'ORF mineur s'étendant du nucléotide 4204 au nucléotide 3875, voire même du nucléotide 4227 au nucléotide 3875 ;
- soit incluse dans la région de la séquence de la figure 1 qui contient la sous-séquence GACA incluse dans l'élément GRE hautement conservé au niveau du nucléotide 6367, cette sous-séquence GACA faisant alors partie de ladite séquence ;
- soit contenue dans la séquence de la figure 1 et contenant elle-même l'une des séquences suivantes :
Consensus : GGTACANNNTGTTCT
HPV39 (NCR) : TCTACATTTTATACT
HPV39 (L1) : GGGACAGTATGTTCT ;
- soit incluse dans l'un des gènes suivants de HPV39 : E1 (nucléotide 922 à nucléotide 2868), E2 (nucléotide 2780 à nucléotide 3907), E4 (nucléotide 3393 à nucléotide 3674), E6 (nucléotide 44 à nucléotide 580) - E7 (nucléotide 493 à nucléotide 918), L1 (nucléotide 5610 à nucléotide 7157), L2 (nucléotide 4172 à nucléotide 5659), soit dans la région intergénique NC.

2. ADN selon la revendication 1, caractérisé en ce que sa séquence correspond à celle du grand ORF de 1,3 kb s'étendant du nucléotide 2050 au nucléotide 776 de la figure 1 ou à tout fragment contenu dans cette séquence ou qui en serait dérivé, ce fragment comportant au moins 15 nucléotides.

3. ADN selon la revendication 1, caractérisé en ce que sa séquence correspond à celle de ORF mineur s'étendant du nucléotide 4204 au nucléotide 3875 et voire même du nucléotide 4227 au nucléotide 3875 ou des séquences issues ou dérivées de ces dernières.

4. ADN selon la revendication 1 caractérisé en ce qu'elle contient au moins 15 nucléotides et qu'elle contient la séquence GACA incluse dans l'élément GRE hautement conservé au niveau du nucléotide 6367.

5. ADN selon la revendication 1, caractérisé en que la séquence contient l'une des séquences suivantes:
Consensus : GGTACANNNTGTTCT
HPV39 (NCR) : TCTACATTTTATACT
HPV39 (L1) : GGGACAGTATGTTCT

6. ADN selon la revendication 1, caractérisé en ce que ledit fragment correspond à l'un des gènes suivants de HPV39 : E1 (nucléotide 922 à nucléotide 2868), E2 (nucléotide 2780 à nucléotide 3907), E4 (nucléotide 3393 à nucléotide 3674), E6 (nucléotide 44 à nucléotide 580)-E7 (nucléotide 493 à nucléotide 918), L1 (nucléotide 5610 à nucléotide 7157), L2 (nucléotide 4172 à nucléotide 5659) ou à tout ou partie de la région intergénique NC.

7. Sonde d'hybridation constituée par un ADN dérivé de l'ADN selon l'une quelconque des revendications 1 à 6.

8. Polypeptide correspondant au produit d'expression de l'un des gènes de structure de l'un des ADNs de papillomavirus, selon l'une quelconque des revendications 1 à 6.

9. Polypeptide selon la revendication 8, caractérisé en ce qu'il consiste plus particulièrement en un produit d'expression de la séquence L2 du génome du papillomavirus HPV39.

10. Anticorps dirigés contre le polypeptide selon la revendication 6 ou la revendication 8.

11. Procédé de détection in vitro sur un échantillon biologique à tester, d'une infection par un papillomavirus du type HPV39, caractérisé par la mise en contact d'une sonde correspondante, telle que définie dans la revendication 7, avec les acides nucléiques de cet échantillon, le cas échéant préalablement rendus accessibles à la sonde, de préférence dans des conditions d'hybridation stringente, et par la détection de l'hybride formé entre l'ADN viral recherché, éventuellement présent dans l'échantillon, et ladite sonde.

12. Procédé selon la revendication 11, caractérisé en ce que le diagnostic in vitro est orienté vers la détection de néoplasies génitales, notamment de cancers cervicaux, vulvaires ou péniens.

13. Application des anticorps selon la revendication 8 au diagnostic in vitro d'une infection par un papillomavirus d'un type susceptible d'induire des néoplasies génitales et des cancers du col de l'utérus, caractérisée par la mise en contact d'un anticorps, tel que défini dans la revendication 10, avec un échantillon biologique provenant de la personne soumise au diagnostic in vitro, et par la détection du complexe antigène-anticorps formé.

## Claims

1. DNA of an HPV39 papillomavirus, characterized either by the nucleotide sequence of Figure 1, or by a portion of this sequence but containing at least fifteen nucleotides, or by a sequence enabling this DNA to hybridize with HPV39 DNA under stringent conditions, the abovementioned portion of sequence being:
- either included in that of the large, 1.3-kb ORF extending from nucleotide 2050 to nucleotide 776 of Figure 1;
- or included in that of the minor ORF extending from nucleotide 4204 to nucleotide 3875, or even from nucleotide 4227 to nucleotide 3875;
- or included in the region of the sequence of Figure 1 which contains the subsequence GACA included in the GRE element which is highly conserved at nucleotide 6367, this subsequence GACA then forming part of the said sequence;
- or contained in the sequence of Figure 1 and itself containing one of the following sequences:
Consensus: GGTACANNNTGTTCT
HPV39 (NCR): TCTACATTTTATACT
HPV39 (L1): GGGACAGTATGTTCT;
- or included in one of the following genes of HPV39: E1 (nucleotide 922 to nucleotide 2868), E2 (nucleotide 2780 to nucleotide 3907), E4 (nucleotide 3393 to nucleotide 3674), E6 (nucleotide 44 to nucleotide 580) - E7 (nucleotide 493 to nucleotide 918), L1 (nucleotide 5610 to nucleotide 7157), L2 (nucleotide 4172 to nucleotide 5659), on in the intergenic NC region.

2. DNA according to Claim 1, characterized in that its sequence corresponds to that of the large, 1.3kb ORF extending from nucleotide 2050 to nucleotide 776 of Figure 1 or to any fragment contained in this sequence or which might be derived therefrom, this fragment possessing at least 15 nucleotides.

3. DNA according to Claim 1, characterized in that its sequence corresponds to that of the minor ORF extending from nucleotide 4204 to nucleotide 3875, and even from nucleotide 4227 to nucleotide 3875, or of sequences originating or derived from these latter.

4. DNA according to Claim 1, characterized in that it contains at least 15 nucleotides and in that it contains the sequence GACA included in the GRE element which is highly conserved at nucleotide 6367.

5. DNA according to Claim 1, characterized in that the sequence contains one of the following sequences:
Consensus : GGTACANNNTGTTCT
HPV39 (NCR) : TCTACATTTTATACT
HPV39 (L1) : GGGACAGTATGTTCT

6. DNA according to Claim 1, characterized in that the said fragment corresponds to one of the following genes of HPV39: E1 (nucleotide 922 to nucleotide 2868), E2 (nucleotide 2780 to nucleotide 3907), E4 (nucleotide 3393 to nucleotide 3674), E6 (nucleotide 44 to nucleotide 580) - E7 (nucleotide 493 to nucleotide 918), L1 (nucleotide 5610 to nucleotide 7157), L2 (nucleotide 4172 to nucleotide 5659), or to all or part of the intergenic NC region.

7. Hybridization probe consisting of a DNA derived from the DNA according to any one of Claims 1 to 6.

8. Polypeptide corresponding to the expression product of one of the structural genes of one of the papillomavirus DNAs according to any one of Claims 1 to 6.

9. Polypeptide according to Claim 8, characterized in that it consists more especially of an expression product of the L2 sequence of the HPV39 papillomavirus genome.

10. Antibodies directed against the polypeptide according to Claim 6 or Claim 8.

11. Method for the in vitro detection of a type HPV39 papillomavirus infection on a biological test sample, characterized by the bringing of a corresponding probe as defined in Claim 7 into contact with the nucleic acids of this sample, where appropriate previously rendered accessible to the probe, preferably under stringent hybridization conditions, and by the detection of the hybrid formed between the viral DNA sought, possibly present in the sample, and the said probe.

12. Method according to Claim 11, characterized in that the in vitro diagnosis is directed towards the detection of genital neoplasia, in particular of cervical, vulvar or penile cancers.

13. Use of the antibodies according to Claim 8 in the in vitro diagnosis of an infection by a papillomavirus of a type capable of inducing genital neoplasia and cancers of the neck of the uterus, characterized by the bringing of an antibody as defined in Claim 10 into contact with a biological sample originating from the person undergoing the in vitro diagnosis, and by the detection of the antigen-antibody complex formed.

## Patentansprüche

1. DNS eines Papillomavirus HPV39 gekennzeichnet durch die Nukleotidsequenz von Figur 1 oder durch einen Teil dieser Sequenz, die aber mindestens 15 Nukleotide enthält, oder auch durch eine Sequenz, die dieser DNS erlaubt, mit der DNS von HPV39 unter bestimmten Bedingungen zu hybridisieren, wobei der genannte Teil der Sequenz:
- in der des großen ORF von 1,3 kb enthalten ist, der sich von Nukleotid 2050 bis Nukleotid 776 in Figur 1 erstreckt;
- oder in der des kleinen ORF enthalten ist, der sich von Nukleotid 4204 bis Nukleotid 3875 erstreckt ja sogar von Nukleotid 4227 bis Nukleotid 3875;
- oder in der Region der Sequenz in Figur 1 enthalten ist, die die Untersequenz GACA im konservierten Element GRE auf Höhe des Nukleotids 6367 enthält, wobei die Untersequenz GACA Teil der genannten Sequenz ist;
- oder in der Sequenz in Figur 1 enthalten ist und selbst eine der folgenden Sequenzen enthält:
Consensus: GGTACANNNTGTTCT
HPV39 (NCR): TCTACATTTTATACT
HPV39 (L1): GGGACAGTATGTTCT;
- oder in einem der folgenden Gene von HPV39 enthalten ist: E1 (Nukleotid 922 bis Nukleotid 2868), E2 (Nukleotid 2780 bis Nukleotid 3907), E4 (Nukleotid 3393 bis Nukleotid 3674), E6 (Nukleotid 44 bis Nukleotid 580) - E7 (Nukleotid 493 bis Nukleotid 918), L1 (Nukleotid 5610 bis Nukleotid 7157), L2 (Nukleotid 4172 bis Nukleotid 5659) oder in der intergenen Region NC.

2. DNS nach Anspruch 1, dadurch gekennzeichnet, daß ihre Sequenz der des großen ORF von 1,3 kb entspricht, der sich von Nukleotid 2050 bis Nukleotid 776 in Figur 1 erstreckt oder irgendeinem Fragment, das in dieser Sequenz enthalten oder davon abgeleitet ist, wobei dieses Fragment mindestens 15 Nukleotide umfaßt.

3. DNS nach Anspruch 1, dadurch gekennzeichnet, daß ihre Sequenz der des kleinen ORF entspricht, der sich von Nukleotid 4204 bis Nukleotid 3875 und sogar von Nukleotid 4227 bis Nukleotid 3875 erstreckt oder Sequenzen, die von diesen letzteren abstammen oder abgeleitet sind.

4. DNS nach Anspruch 1, dadurch gekennzeichnet, daß sie mindestens 15 Nukleotide enthält und daß sie die Sequenz GACA im konservierten Element GRE auf Höhe des Nukleotids 6367 aufweist.

5. DNS nach Anspruch 1, dadurch gekennzeichnet, daß die Sequenz eine der folgenden Sequenzen enthält:
Consensus: GGTACANNNTGTTCT
HPV39 (NCR): TCTACATTTTATACT
HPV39 (L1): GGGACAGTATGTTCT

6. DNS nach Anspruch 1, dadurch gekennzeichnet, daß das genannte Fragment einem der folgenden Gene von HPV39 entspricht: E1 (Nukleotid 922 bis Nukleotid 2868), E2 (Nukleotid 2780 bis Nukleotid 3907), E4 (Nukleotid 3393 bis Nukleotid 3674), E6 (Nukleotid 44 bis Nukleotid 580) - E7 (Nukleotid 493 bis Nukleotid 918), L1 (Nukleotid 5610 bis Nukleotid 7157), L2 (Nukleotid 4172 bis Nukleotid 5659) oder die ganze oder ein Teil der intergenen Region NC.

7. Hybridisierungssonde gebildet von einer DNS, die von der DNS nach einem der Ansprüche 1 bis 6 abgeleitet ist.

8. Polypeptid entsprechend dem Produkt der Expression von einem der Gene der Struktur von einer der Papillomavirus-DNS nach einem der Ansprüche 1 bis 6.

9. Polypeptid nach Anspruch 8, dadurch gekennzeichnet, daß es insbesondere in einem Produkt der Expression der Sequenz L2 des Genoms von Papillomavirus HPV39 besteht.

10. Antikörper, die gegen das Polypeptid nach Anspruch 6 oder nach Anspruch 8 gerichtet sind.

11. Verfahren zum Nachweis einer Infektion durch einen Papillomavirus vom Typ HPV39 auf einer zu prüfenden biologischen Probe in vitro, gekennzeichnet durch in Kontakt bringen einer entsprechenden Sonde, wie sie in Anspruch 7 definiert ist, mit den Nukleinsäuren dieser Probe, die gegebenenfalls zuvor für die Sonde zugänglich gemacht wurden, bevorzugt unter bestimmten Hybridisierungsbedingungen und durch Nachweis des zwischen der gesuchten Virus-DNS, die eventuell in der Probe vorhanden ist, und der genannten Sonde gebildeten Hybrids.

12. Verfahren nach Anspruch 11, dadurch gekennzeichnet, daß die Diagnostik in vitro auf den Nachweis von Neoplasien im Genitalbereich, insbesondere von Krebsen der Cervix, der Vulva und des Penis gerichtet ist.

13. Anwendung von Antikörpern nach Anspruch 8 zur Diagnose in vitro einer Infektion durch einen Papillomavirus eines Typs, der geeignet ist, Neoplasien im Genitalbereich und Krebse des Gebärmutterhalses zu induzieren, gekennzeichnet durch in Kontakt bringen eines Antikörpers, wie in Anspruch 10 definiert, mit einer biologischen Probe, die von einer Person stammt, die sich der in vitro Diagnose unterzieht und durch Nachweis des gebildeten Antigen-Antikörper-Komplexes.
